# EUROPEAN PATENT APPLICATION

(11) **EP 4 725 918 A1**
(43) Date of publication of application: **15.04.2026**
(21) Application number: 23940667.1
(22) Date of filing: 07.06.2023
(51) Int. Cl.: C02F 11/00, C02F 11/04

(54) **METHANE FERMENTATION METHOD AND METHOD FOR PRODUCING METHANE FERMENTATION ACCELERATOR**

(71) Applicant: Biofuel Technology Research Co., Ltd., Hiroshima-shi, Hiroshima 731-0103 (JP)
(72) Inventor: KAJIMA, Hiroshi, Hiroshima-shi, Hiroshima 731-0103 (JP); TACHIBANA, Mineo, Hiroshima-shi, Hiroshima 731-0103 (JP); ABE, Takeshi, Tokyo 104-8307 (JP); JONO, Koji, Tokyo 104-8307 (JP)
(74) Representative: Fink Numrich Patentanwälte PartmbB
(86) International application number: PCT/JP2023/021180
(87) International publication number: WO 2024/252565

(57) **Abstract**

A methane fermentation method comprising charging organic waste into a methane fermentation system to produce biogas containing methane, wherein a methane fermentation accelerator is used in combination with the organic waste, wherein the methane fermentation accelerator contains glycerin, wherein the methane fermentation accelerator contains an n-hexane extract of 10,000 mg/kg or less. The present invention provides a methane fermentation method that utilizes glycerin to efficiently produce methane-containing biogas from organic waste and also provides a methane fermentation accelerator that can accelerate methane fermentation.

## Description

### Technical Field

The present invention relates to a methane fermentation method in which organic waste is charged into a methane fermentation system to produce methane-containing biogas, and particularly to a method in which a specific methane fermentation accelerator is used in combination with the organic waste. The present invention also relates to a method for producing a methane fermentation accelerator used in the above methane fermentation method.

### Background Art

Organic waste substances, such as food waste, sludge generated from sewage treatment plants, and livestock manure, have conventionally been considered biomass resources, and energy has been recovered from them. Methane fermentation is one of the most effective means for recovering energy from such organic waste.

In the meantime, from the perspective of preventing global warming, the development of fuels that replace conventional fossil fuels has been promoted, including those reducing the generation of carbon dioxide and leading to the recycling of resources. As one of them, biodiesel fuels whose raw materials are plant oil, waste cooking oil, and the like are attracting attention. The mainstream method of synthesizing a biodiesel fuel is a method in which the synthesis is performed by a transesterification reaction using animal/plant oil/fat and/or oil/fat such as waste cooking oil and a monohydric alcohol as the raw materials and an alkaline substance as the catalyst (e.g., Non-Patent Document 1). In this synthetic reaction, a glycerin-containing by-product (waste glycerin) is also generated.

Examples of the methods for industrially producing a free fatty acid from oil/fat include high-temperature and high-pressure decomposition methods and enzymatic decomposition methods, all of which hydrolyze oil/fat derived from animal/plant and the like to release a fatty acid. Also in such hydrolysis, a glycerin-containing by-product is generated.

Glycerin-containing waste contains a large amount of impurities such as a catalyst and unreacted oil/fat. Accordingly, while glycerin itself may be used as a raw material for pharmaceuticals, cosmetics, and the like, it is necessary to purify the above-described glycerin-containing waste at a large cost when using it as a raw material for pharmaceuticals, cosmetics, and the like, which may not be practical. Therefore, glycerin-containing waste has often been discarded as industrial waste.

Among others, methods have been proposed in which glycerin-containing waste, such as waste glycerin, is mixed with food waste or the like and used as a raw material for the above-described methane fermentation (e.g., Patent Documents 1 and 2).

### Prior Art Documents

### Patent Documents

Patent Document 1: JP2006-348191A
Patent Document 2: JP2005-279411A

### Non-Patent Documents

Non-Patent Document 1: Journal of the Japan Institute of Marine Engineering, 2012, Vol. 47, No. 1, pp. 45-50

### Summary of the Invention

### Problems to be solved by the Invention

It is, however, known that the above glycerin-containing waste has an effect of inhibiting methane fermentation, and it cannot be said that the glycerin-containing waste is useful as a raw material for methane fermentation. As such, only very small amounts can be added as a raw material for methane fermentation, and it cannot be said to be sufficient from the perspective of effective utilization of glycerin-containing waste.

The present invention has been made in view of the above problems, and objects of the present invention include providing a methane fermentation method that utilizes glycerin to efficiently produce methane-containing biogas from organic waste and also providing a methane fermentation accelerator that can accelerate methane fermentation.

### Means for solving the Problems

The present inventors have found that a specific composition containing glycerin can accelerate methane fermentation rather than inhibit it during methane fermentation using organic waste as a raw material, and have accomplished the present invention.

Specifically, the present invention is as follows.
[1] A methane fermentation method comprising charging organic waste into a methane fermentation system to produce biogas containing methane,
   wherein a methane fermentation accelerator is used in combination with the organic waste,
   wherein the methane fermentation accelerator contains glycerin, wherein the methane fermentation accelerator contains an n-hexane extract of 10,000 mg/kg or less.
[2] The methane fermentation method according to [1], wherein the glycerin content in the methane fermentation accelerator is 600,000 mg/kg or more.
[3] The methane fermentation method according to [1] or [2] , wherein the methane fermentation accelerator is used so that a ratio of COD_{Cr} in the methane fermentation accelerator to COD_{Cr} in the organic waste is 10:1 to 1:10.
[4] A method for producing a methane fermentation accelerator to be combined with organic waste and charged into a methane fermentation system, comprising:
   a first separation step of mixing a raw material containing at least one of glycerin and a fatty acid glycerin ester with an inorganic acid and separating a first oil component and a first glycerin-containing liquid;
   a neutralization step of neutralizing the first glycerin-containing liquid with an alkaline substance; and
   a second separation step of separating a second oil component and a precipitated inorganic salt from the neutralized first glycerin-containing liquid,
   wherein the obtained methane fermentation accelerator contains glycerin.
[5] The method for producing a methane fermentation accelerator according to [4], further comprising
   an alcohol removal step of removing monohydric alcohol after the second separation step.
[6] The method for producing a methane fermentation accelerator according to [4] or [5], wherein pH of a mixed liquid of the raw material and the inorganic acid in the first separation step is 3 or less.
[7] The method for producing a methane fermentation accelerator according to any one of [4] to [6], wherein the first glycerin-containing liquid is neutralized to pH of 4 to 8 in the neutralization step.
[8] The method for producing a methane fermentation accelerator according to any one of [4] to [7], wherein the methane fermentation accelerator contains an n-hexane extract of 10,000 mg/kg or less.
[9] The method for producing a methane fermentation accelerator according to any one of [4] to [8], wherein the glycerin content in the methane fermentation accelerator is 600,000 mg/kg or more.

### Advantageous Effect of the Invention

According to the methane fermentation method of the present invention, biogas containing methane can be efficiently produced through methane fermentation using organic waste as a raw material. Furthermore, the methane fermentation accelerator obtained in the present invention can accelerate methane fermentation using organic waste as a raw material.

### Brief Description of the Drawings

[FIG. 1] FIG. 1 is a diagram illustrating a flow of the method for producing a methane fermentation accelerator according to an embodiment of the present invention.
[FIG. 2] FIG. 2 is a diagram illustrating a flow of an esterification step (second esterification step) included in a preferred embodiment of the present invention.
[FIG. 3] FIG. 3 is a schematic diagram illustrating an experiment apparatus used in examples.

### Embodiments for Carrying out the Invention

Hereinafter, one or more embodiments of the present invention will be described.

### [Methane Fermentation Method and Methane Fermentation Accelerator]

The methane fermentation method according to an embodiment of the present invention involves charging organic waste into a methane fermentation system to produce methane-containing biogas, in which a glycerin-containing methane fermentation accelerator is used in combination with the organic waste.

### (1) Methane Fermentation System

As used in the present specification, the "methane fermentation system" refers to an organic mixture colonized by various anaerobic microorganisms, primarily methanogens (such as hydrolytic bacteria, acid-producing bacteria, and methanogens), capable of decomposing organic matter and fermenting it into methane.

The methane fermentation system may be obtained by anaerobically treating organic waste, such as sewage sludge or animal manure, to colonize the above anaerobic microorganisms, or a portion of the methane fermentation system may be collected and used from another stably operating methane fermenter. Such a methane fermentation system contains a certain amount of essential elements for performing methane fermentation, and a methane fermentation reaction can occur on its own, but in a typical methane fermentation method, organic waste, as described below, is also added as a raw material.

### (2) Organic Waste

As used in the present specification, the "organic waste" refers to waste containing organic matter, such as organic sludge, livestock manure, food waste, food waste, rice straw, and grass clippings, discarded from sewage treatment facilities, sludge recycling centers, sewage treatment facilities, food factories, etc., which has traditionally been used as a raw material for methane fermentation.

The properties of the organic waste used in the present embodiment are not particularly limited, but COD_{Cr} may be, for example, 10,000 mg/kg or more in an embodiment or 100,000 mg/kg or more in another embodiment.

Here, COD (chemical oxygen demand) is an index that represents the amount of organic matter in a composition as an "oxygen consumption when decomposed by an oxidant," and there are several types depending on the type of oxidant and reaction conditions used. In the present embodiment, "COD_{Cr}," which uses potassium dichromate as the oxidant, is used as the COD index. COD_{Cr} can be measured, for example, in accordance with JIS K0102.

The organic waste used in the present embodiment can be suitably used even when it has a low COD_{Cr} decomposition ratio when used alone in methane fermentation. Here, the COD_{Cr} decomposition ratio is a value calculated by dividing the decomposition COD_{Cr} by the COD_{Cr} of the input raw material. The decomposition COD_{Cr} is a value calculated from the amount of methane generated, using the formula: amount of methane generated/decomposition COD_{Cr} = 0.35 n-m³/kg·COD_{Cr}. In other words, it can be said that the COD_{Cr} decomposition ratio is an index that represents the utilization efficiency of the carbon source in methane fermentation.

Organic waste with a low COD_{Cr} decomposition ratio when used alone has little utility value as a raw material for methane fermentation. The residue remaining after the methane fermentation reaction in a methane fermentation system is called digestate, and requires wastewater treatment, but the use of a raw material with a low COD_{Cr} decomposition ratio places a heavy burden on the wastewater treatment, resulting in high costs.

In contrast, according to a preferred aspect of the present embodiment, when used in combination with the above methane fermentation accelerator, for example, the COD_{Cr} decomposition ratio can be improved. This allows organic waste, which has traditionally been considered to have little utility value, to be suitably used for methane fermentation. Furthermore, wastewater treatment for the digestate is also easier.

The organic waste used in the present embodiment may have a glycerin content of 100,000 mg/kg or less in an embodiment or 10,000 mg/kg or less in another embodiment, or may contain no glycerin at all. This is because organic waste containing relatively high concentrations of glycerin often also contains high concentrations of oil components, which can reduce the efficiency of methane fermentation, and producing a methane fermentation accelerator using the method described later and combining it with other organic waste for use increases the efficiency of methane fermentation, rather than using it directly as a carbon source.

### (3) Methane Fermentation Accelerator

The methane fermentation accelerator used in the present embodiment contains glycerin. When used in combination with the above-described organic waste during methane fermentation, the glycerin-containing methane fermentation accelerator can accelerate methane fermentation.

Glycerin is a trihydric alcohol whose carbon number is three. and is converted into acetic acid, propionic acid, and other acids by anaerobic microorganisms (e.g., acid-producing bacteria) that colonize methane fermentation systems, and these acids serve as raw materials for methane fermentation reactions using methanogens. That is, glycerin alone can be useful as a raw material for methane fermentation. Fortunately, however, as will be disclosed in the examples described later, the use of a methane fermentation accelerator containing glycerin in combination with organic waste accelerates methane fermentation reactions, as compared with not only when the organic waste is used alone as a raw material, but also when the methane fermentation accelerator is used alone.

While such effects of the present embodiment are not limited to a specific mechanism of action, for example, because glycerin is a carbon source that is easily utilized not only by methanogens but also by other anaerobic microorganisms (e.g., hydrolytic bacteria, acid-producing bacteria, etc.) that have colonized the methane fermentation system, it is presumed that not only methanogens but also other anaerobic microorganisms are activated, optimizing the overall balance of the methane fermentation system. Then, optimizing the balance of the methane fermentation system makes it possible to efficiently utilize not only glycerin (methane fermentation accelerator) but also the organic waste in the methane fermentation reaction, thereby presumably accelerating the methane fermentation reaction as a whole.

Note, however, that the methane fermentation accelerating effect according to the present embodiment is not limited to that based on such a mechanism of action.

The glycerin content in the methane fermentation accelerator is preferably 600,000 mg/kg or more, more preferably 700,000 mg/kg or more, and particularly preferably 900,000 mg/kg or more. There is no particular upper limit to the glycerin content in the methane fermentation accelerator. For example, it may be 100% (1,000,000 mg/kg), but such high purity is not particularly required.

The methane fermentation accelerator used in the present embodiment preferably contains an n-hexane extract (n-Hex) of 10,000 mg/kg or less, more preferably 5,000 mg/kg or less, and particularly preferably 2,000 mg/kg or less. Here, n-Hex is a general term for non-volatile substances extracted by the organic solvent n-hexane, and is used as an index representing the amount of "oil components and the like" in a composition. The n-Hex can be measured using the extraction/gravimetric method as defined in Public Notice No. 64 of the Ministry of the Environment in 1974, Appendix Table 4. The methane fermentation accelerator of the present embodiment has a small n-Hex value, or in other words, the oil components are removed, and it is thereby possible to improve the efficiency of methane fermentation.

In addition to the above, examples of methane fermentation accelerators used in the present embodiment include those having the following properties.

The COD_{Cr} of the methane fermentation accelerator is preferably 730,000 mg/kg or more, more preferably 850,000 mg/kg or more, and particularly preferably 1,100,000 mg/kg or more. The upper limit of the COD_{Cr} of the methane fermentation accelerator is not particularly limited, and for example, it may be the COD_{Cr} (1,220,000 mg/kg) when the glycerin content is 100%, but such a high COD_{Cr} is not particularly required.

In the present embodiment, the methane fermentation accelerator is used in combination with the above-described organic waste.

In this context, the concept of "used in combination" encompasses not only a form in which the methane fermentation accelerator and organic waste are mixed and charged into the methane fermentation system, but also a form in which the methane fermentation accelerator and organic waste are charged into the methane fermentation system separately without being mixed. However, when charging the methane fermentation accelerator and organic waste into the methane fermentation system separately, from the perspective of effectively exhibiting the methane fermentation acceleration effect, it is preferred to charge the methane fermentation accelerator and organic waste simultaneously, or to start charging one of them within a predetermined time (e.g., within 24 hours) after the completion of charge with the other.

The usage ratio of the methane fermentation accelerator and the organic waste in combination can be adjusted appropriately from the perspective of obtaining the effect of accelerating the methane fermentation, but for example, it is preferred to use the above methane fermentation accelerator so that the ratio of the COD_{Cr} of the methane fermentation accelerator to the COD_{Cr} of the organic waste (methane fermentation accelerator:organic waste) is between 10:1 and 1:10. When the usage ratio of the methane fermentation accelerator to the organic waste is within the above range, the effect of accelerating the methane fermentation is more effectively achieved.

### (4) Auxiliary Raw Materials

In the present embodiment, in addition to the above-described organic waste and methane fermentation accelerator, chemical substances containing phosphorus and/or nitrogen may be used as auxiliary raw materials as appropriate, such as phosphoric acid, phosphate salts, ammonia, and ammonium salts, for example, specifically, ammonium phosphate, ammonium chloride, ammonium sulfate, phosphoric acid, potassium phosphate, magnesium phosphate, etc. These auxiliary raw materials are charged into the methane fermentation system along with the organic waste and methane fermentation accelerator.

### (5) Methane Fermentation Reaction

The methane fermentation reaction typically involves placing a methane fermentation system colonized by various anaerobic microorganisms, primarily methanogens, in a methane fermenter as a reaction vessel, and maintaining anaerobic conditions as the reaction conditions. The above methane fermentation system is then charged with organic waste as the raw material as well as auxiliary materials, and other organic matter/essential elements that accelerate the methane fermentation, and they are in a mixture form. The methane fermenter is preferably equipped with an agitator to ensure uniform dispersion of the methane fermentation system, raw materials, methane-producing bacteria, and other components, allowing the methane fermentation reaction to proceed.

The methane fermentation method of the present embodiment is characterized by the use of the above-described methane fermentation accelerator in combination with organic waste, which has conventionally used as a raw material for methane fermentation, but other aspects of the method are similar to those of conventional methane fermentation, allowing existing methane fermentation equipment and the like to be used without any modification.

In the methane fermenter, the above-described mixture is decomposed by the anaerobic microorganisms, and methane-containing biogas and digestate are generated as the methane fermentation reaction progresses. Specifically, in the methane fermenter, the biogas mainly composed of methane gas generated by methane fermentation stays in the uppermost cavity of the fermenter, and the digestate is stored in the lower part.

In the present embodiment, any of medium-temperature fermentation performed while keeping the inside of the fermenter at around 37°C and high-temperature fermentation performed while maintaining the temperature at around 55°C may be adopted, and it is preferred to carry out the methane fermentation while stirring the content in an anaerobic atmosphere.

The methane fermentation reaction performed in the present embodiment may be a batch type, in which all raw materials are charged into the methane fermenter before the reaction begins and the generated biogas is collected as needed, or alternatively, it may be a continuous type, in which raw materials are continuously charged into the methane fermenter and the generated biogas is continuously collected. In methane fermentation, the generated biogas is easily separated from the digestate and can be easily recovered continuously, so a continuous system can also be suitably used.

When the methane fermentation is carried out in a continuous scheme, it is preferred to appropriately adjust the input amount or the like of the organic waste and methane fermentation accelerator so that the COD_{Cr} load in the methane fermenter falls within a range of 2 to 20 kg/m³·day. More preferably, the COD_{Cr} load in the methane fermenter is controlled within a range of 5 to 10 kg/m³·day. If the COD_{Cr} load is unduly large, the methane fermentation is less likely to progress promptly. By adjusting the inside of the fermenter to the range of the COD_{Cr} load as described above, it will be easy to maintain the rate of methane fermentation. The COD_{Cr} load can be adjusted by the input amounts of methane fermentation raw materials, such as organic waste and methane fermentation accelerator, the dilution amount, and flow rate.

From the perspective of efficiently progressing the methane fermentation reaction, it is preferred to adjust the total nitrogen (T-N) concentration in the methane fermenter to 100 to 10,000 mg/L in an embodiment or 500 to 5,000 mg/L in another embodiment. While the nitrogen components contained in the methane fermentation raw material are used for bacterial synthesis, the decomposition product ammonia may inhibit methane fermentation, so it is preferred to perform adjustment with the mixing ratio or dilution. By adjusting the T-N to be within the above range, the methane fermentation will proceed smoothly and the amount of methane produced will increase. The T-N in the methane fermenter can be adjusted with the input amount of organic waste, which is the primary raw material, and auxiliary raw materials.

Total nitrogen (T-N) indicates the total amount of nitrogen compounds contained in a composition and can be measured in accordance with the "Kjeldahl Nitrogen Method" in Section 18-1 of Chapter 1, Volume 5 of the Sewage Testing Methods.

The total phosphorus (T-P) in the methane fermenter can be adjusted to 100 mg/L or more. The total phosphorus (T-P) is the total amount of phosphorus compounds contained in the composition and can be measured in accordance with JIS K0102-46.3.2, "Nitric Acid-Perchloric Acid Decomposition Method."

The methane-containing biogas generated in the methane fermenter is appropriately recovered and used not only as the fuel for power generation and the like but also for various applications as high-purity methane gas after purification.

### [Method for Producing Methane Fermentation Accelerator]

The methane fermentation accelerator according to the above-described embodiment can be used without any particular limitation, provided that it satisfies the above requirements. However, the production method described below is particularly suitable, as it allows for inexpensive production of a methane fermentation accelerator that satisfies above requirements from industrial waste, etc.

Specifically, the method for producing a methane fermentation accelerator according to an embodiment of the present invention includes: a first separation step of mixing a raw material containing at least one of glycerin and a fatty acid glycerin ester with an inorganic acid and separating a first oil component and a first glycerin-containing liquid; a neutralization step of neutralizing the first glycerin-containing liquid with an alkaline substance; and a second separation step of separating a second oil component and a precipitated inorganic salt from the neutralized first glycerin-containing liquid.

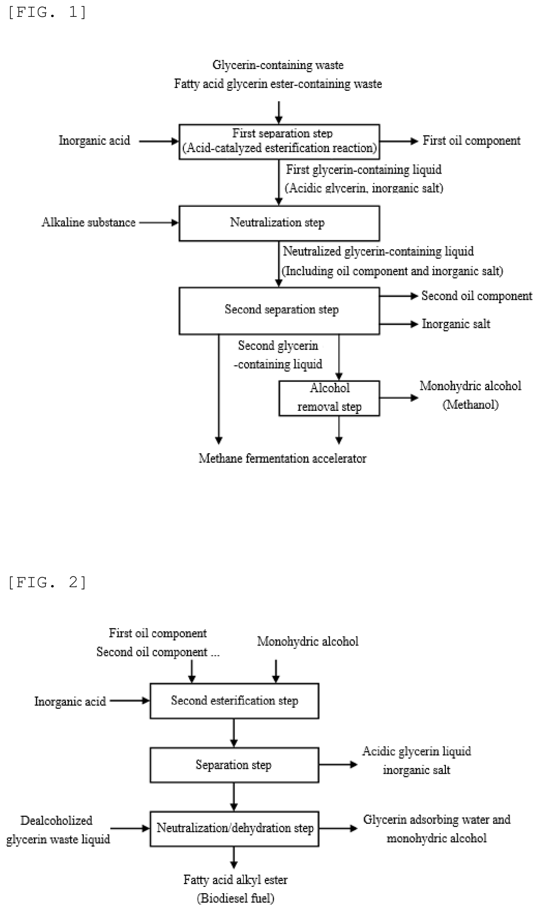

FIG. 1 is a diagram illustrating a flow in a particularly preferred embodiment of the method for producing a methane fermentation accelerator according to the present embodiment. FIG. 1 illustrates a method of obtaining a methane fermentation accelerator through a first separation step of mixing an inorganic acid to a raw material that contains a glycerin-containing waste or a fatty acid glycerin ester-containing waste to separate and remove a first oil component, followed by a neutralization step for a first glycerin-containing liquid and a second separation step of separating and removing a second oil and the inorganic salt from the neutralized glycerin-containing liquid, followed by an alcohol removal step of separating and removing monohydric alcohol from the second glycerin-containing liquid. The alcohol removal step is an optional step, and the second glycerin-containing liquid may be used as a methane fermentation accelerator without through the alcohol removal step.

### (6) Raw Material for Methane Fermentation Accelerator

The raw material for the methane fermentation accelerator used in the present embodiment is not particularly limited, provided that it contains at least one of glycerin and a fatty acid glycerin ester.

Examples of the raw material containing glycerin include wastes that contain glycerin.

On the other hand, raw materials containing a fatty acid glycerin ester are also preferably used because these generate glycerin by an acid-catalyzed transesterification reaction or the like in the first separation step, which will be described later. When performing the esterification step (second esterification step), which will be described later, it is preferred to use a raw material containing a fatty acid glycerin ester as the raw material for the methane fermentation accelerator.

In the present specification, the term "fatty acid glycerin ester" refers to an ester of a fatty acid and glycerin, and is a term that encompasses triglycerides, diglycerides, and monoglycerides.

The glycerin-containing waste and the fatty acid glycerin ester-containing waste will be described below in some detail.

### (6-1) Glycerin-containing waste

Examples of the glycerin-containing waste used in the present embodiment include waste glycerin produced as a by-product in the process of producing a biodiesel fuel, a glycerin waste liquid produced as a by-product in the step of producing a free fatty acid, sweet water, and washing wastewater of a fatty acid alkyl ester.

Here, the glycerin waste liquid produced as a by-product in the step of producing a free fatty acid refers to waste produced as a by-product when hydrolyzing animal/plant oil/fat to produce a free fatty acid. Examples of the method for producing a free fatty acid by hydrolysis include a high-temperature and high-pressure decomposition method and an enzymatic decomposition method. The glycerin waste liquid produced as a by-product in such a production step contains unreacted oil/fat, partially hydrolyzed oil/fat, and the like in addition to glycerin.

The sweet water is a by-product in the case of saponifying oil/fat (performing alkaline hydrolysis of oil/fat) to generate a fatty acid salt (e.g., in the process of producing soap or the like) and contains glycerin, water, alkali, and the like.

The washing wastewater of a fatty acid alkyl ester is wastewater generated when a reactant is washed in the process of producing a fatty acid alkyl ester such as a biodiesel fuel. The wastewater contains not only water but also glycerin produced as a by-product in the production reaction of a fatty acid alkyl ester and further contains an unreacted free fatty acid and its salt, a monohydric alcohol, and the like.

The waste glycerin produced as a by-product in the process of producing a biodiesel fuel will then be described in some detail.

The fatty acid alkyl ester to be a biodiesel fuel can be obtained through adding a monohydric alcohol such as methanol and an alkali catalyst such as potassium hydroxide to raw oil/fat such as plant oil and performing a transesterification reaction.

Examples of the raw oil/fat to be used for a biodiesel fuel include plant oils such as rapeseed oil, palm oil, olive oil, sunflower oil, soybean oil, rice oil, and cannabis oil; animal fats such as fish oil, pork fat, and beef and pork fats; and waste cooking oil such as tempura oil.

Examples of the monohydric alcohol for use include methanol, ethanol, 1-propanol, and ethylhexanol, among which methanol and ethanol are preferred, and methanol is particularly preferred.

Examples of the alkali catalyst for use include potassium hydroxide, sodium hydroxide, and calcium oxide, but potassium hydroxide is preferred from the perspectives of precipitation property, ease of reuse, and the like of the salt which is separated and recovered in the present embodiment.

In the above transesterification reaction, the fatty acid glycerin ester contained in the raw oil/fat reacts with the monohydric alcohol to generate a fatty acid alkyl ester and glycerin. The obtained reaction liquid is separated into two liquid phases of a fatty acid alkyl ester phase and a waste glycerin phase, and in the production of a biodiesel fuel, the obtained fatty acid alkyl ester phase is recovered and subjected to washing and other necessary treatment to obtain a biodiesel fuel.

On the other hand, the waste glycerin phase contains not only glycerin at a high concentration but also an unreacted monohydric alcohol (in particular, methanol), unreacted oil/fat (fatty acid glycerin ester), a fatty acid and its salt, an alkali catalyst, and foreign substances derived from the raw oil/fat. The waste glycerin may be liquid waste glycerin or solid waste glycerin, but from the perspectives of workability, handling, and the like, liquid waste glycerin is preferred.

The content of glycerin, a monohydric alcohol, oil/fat, and a fatty acid and its salt in the waste glycerin is not particularly limited, but usually, the content of glycerin is 25 mass% or more and 65 mass% or less, the content of a monohydric alcohol is 2 mass% or more and 20 mass% or less, and the total content of oil/fat and a fatty acid and its salt is 30 mass% or more and 50 mass% or less in many cases with respect to the waste glycerin as a whole.

The pH of waste glycerin is often 9 or more because it contains a large amount of alkali catalyst, and in the present embodiment, the pH may be 9 to 13.

From the perspective of allowing the acid-catalyzed esterification reaction to readily progress in the first separation step between the unreacted oil/fat and the monohydric alcohol contained in the waste glycerin, the water content in the waste glycerin is preferably 5 mass% or less and particularly preferably 3 mass% or less. The water content in the waste glycerin can be appropriately adjusted by heating, pressure reduction, use of a desiccant or the like, permeation through purified glycerin, etc.

Here, glycerin can be a raw material for pharmaceuticals, cosmetics, and the like, but in order to use the glycerin contained in the waste glycerin for such applications, it is necessary to purify it to high purity, which requires a great deal of cost and energy. Thus, the utility value of waste glycerin as glycerin is considerably low, and the waste glycerin has heretofore been difficult to process.

Fortunately, however, according to the present embodiment, waste glycerin can be used as the main raw material for the methane fermentation accelerator, and the environmental load can be reduced from the perspective of effectively utilizing the waste glycerin, which is an industrial waste. Furthermore, because the methane fermentation accelerators produced from waste glycerin use carbon-neutral natural oils as raw materials, the energy produced by methane fermentation, such as biogas, electricity, and heat, can be classified as renewable energy.

In the present embodiment, from the perspective of ease of use in the first separation step, which will be described later, it is preferred to use, among the above-described glycerin-containing wastes, one of the waste glycerin produced as a by-product in the process of producing biodiesel fuel and the glycerin waste liquid produced as a by-product in the step of producing a free fatty acid, and it is particularly preferred to use the waste glycerin produced as a by-product in the process of producing biodiesel fuel.

### (6-2) Fatty acid glycerin ester-containing waste

In the present embodiment, waste that contains a fatty acid glycerin ester can also be used as the raw material for a methane fermentation accelerator. The present embodiment is carried out through the first separation step using an inorganic acid, the neutralization step, and the second separation step, and the acid-catalyzed esterification reaction in the first separation step can therefore increase the yield of glycerin using the waste containing a fatty acid glycerin ester.

Examples of the waste containing a fatty acid glycerin ester include oil/fat that contains as the main component a fatty acid glycerin ester of waste cooking oil, food containing fat/oil that has expired (such as tempura oil, mayonnaise, dressings, butter, cream, or cheese), animal/plant oil, or high acid value oil (such as grease trap oil, sewage oil, gutter oil, or waste liquid treatment recycled oil); and a composition that contains as the main component a fatty acid salt of an oil cake, soap, or the like.

In the present specification, the "main component" means a component having the highest content in the composition (when the component having the highest content is water, a component having the second highest content), and the content is preferably 40 mass% or more and more preferably 50 mass% or more.

Here, the high acid value oil refers to oil/fat having an acid value of 10 mgKOH/g or more, and examples thereof include a free fatty acid in addition to a fatty acid glycerin ester that is the main component of oil/fat. The acid value may be 20 mgKOH/g or more in an embodiment or 50 mgKOH/g or more in another embodiment. The upper limit of the acid value is usually 200 mgKOH/g or less.

The oil cake is a by-product separated from oil/fat (raw oil) in the deacidification step in the purification of plant oil/fat and contains a fatty acid salt, a fatty acid glycerin ester, alkali, water, and the like.

Compositions other than those exemplified above can also be used, provided that they contain fatty acid glycerin esters. For example, waste glycerin, which is a by-product of the biodiesel fuel production process and is exemplified as a glycerin-containing waste, contains unreacted oil/fat (i.e., fatty acid glycerin ester), and therefore can also be used as fatty acid glycerin ester-containing waste.

### (7) First Separation Step

The first separation step is a step of mixing the raw material, which contains at least one of glycerin and a fatty acid glycerin ester, with an inorganic acid and phase-separating a first oil component and a first glycerin-containing liquid.

The oil component separated in this step contains a fatty acid glycerin ester and a free fatty acid in addition to a fatty acid alkyl ester.

When using a raw material that contains glycerin, especially when using waste glycerin, the fatty acid salt contained in the waste glycerin or the like is converted into a free fatty acid by the inorganic acid in this step. The fatty acid and its salt generate a fatty acid alkyl ester by an esterification reaction with an unreacted monohydric alcohol contained in the waste glycerin using the inorganic acid as an acid catalyst.

When using a fatty acid glycerin ester-containing waste as the raw material, a fatty acid alkyl ester and glycerin are generated by a transesterification reaction with the monohydric alcohol. The monohydric alcohol in this case can be added separately, and for example, the monohydric alcohol recovered in the alcohol separation step, which will be described later, can be used. Additionally or alternatively, waste glycerin may be treated at the same time as the fatty acid glycerin ester-containing waste thereby to use an unreacted monohydric alcohol contained in the waste glycerin.

When performing the first separation step under the presence of a monohydric alcohol, this step can also be referred to as an acid-catalyzed esterification step. In comparison with the second esterification reaction, which will be described later, the first separation step may be referred to as a "first esterification step."

Even when the monohydric alcohol is not contained, the fatty acid glycerin ester generates a free fatty acid and glycerin under the presence of an acid in the first separation step. Additionally or alternatively, when the raw material contains a fatty acid salt, the fatty acid salt is converted by the acid into a free fatty acid, which may readily be separated from the glycerin.

Therefore, the present embodiment can be preferably applied even when the raw material contains no monohydric alcohol.

In the present embodiment, various raw materials can be treated at the same time because the first separation step is performed under the presence of an inorganic acid. Moreover, by performing the first separation step, the waste such as waste glycerin, waste cooking oil, or high acid value oil, which contains glycerin and/or a fatty acid glycerin ester, can be effectively utilized, and this can therefore contribute to reduction of the environmental load.

Among these, the high acid value oil has a high acid value of 10 mgKOH/g or more and is therefore difficult to use as the raw material for the transesterification reaction using the previously described alkali catalyst. Fortunately, however, the high acid value oil can also be preferably used as the raw material in the first separation step, which should be also referred to as an acid-catalyzed esterification reaction.

When using waste glycerin, a fatty acid glycerin ester-containing waste, and the like as the raw materials for a methane fermentation accelerator, the fatty acid alkyl ester and free fatty acid produced in the first separation step migrate to the oil phase composed of the first oil component and can therefore be separated from the first glycerin-containing liquid. When the oil phase is recovered, the obtained first oil component (such as a fatty acid alkyl ester or a free fatty acid) is subjected to a further esterification reaction (esterification step described later) and can be finally used as a raw material for a biodiesel fuel or the like.

On the other hand, the first glycerin-containing liquid is acidified by the addition of an inorganic acid. Moreover, the first glycerin-containing liquid may contain an inorganic salt generated from the inorganic acid and an alkali that is contained in the glycerin-containing waste. A part of the inorganic salt may be precipitated, that is, the first glycerin-containing liquid may contain an acidic glycerin phase and the precipitated inorganic salt.

The water content in the raw material which can be used in the first separation step is preferably 10 mass% or less in an embodiment or preferably 5 mass% or less in another embodiment. By using a raw material having a low water content (e.g., waste glycerin or the like having a low water content), it becomes easy to reduce the water content in the reaction liquid, which will be described later. The water content in the raw material can be appropriately adjusted by heating, pressure reduction, use of a desiccant or the like, permeation through purified glycerin, etc.

Examples of the inorganic acid used in the first separation step include concentrated sulfuric acid, phosphoric acid, concentrated nitric acid, and hydrogen chloride, but concentrated sulfuric acid and phosphoric acid having a low water content are preferred, and concentrated sulfuric acid is particularly preferred.

In the first separation step, the pH of the mixed liquid (reaction liquid) of the above raw material and the above inorganic acid is preferably 3 or less and particularly preferably 1 or less. The pH of the reaction liquid can be adjusted by the additive amount of the above inorganic acid.

The water content in the reaction liquid is preferably 10 mass% or less and particularly preferably 0.5 mass% or less. The water content in the reaction liquid can be appropriately adjusted by adjustment of the water content and input amount of each raw material, use of a desiccant in the reaction liquid, etc.

By setting the pH and water content of the reaction liquid within the above ranges, the efficiency of the acid-catalyzed esterification reaction can be increased, and the first oil component and the first glycerin-containing liquid (including the acidic glycerin phase and the inorganic salt) can be well separated.

The temperature of the reaction liquid in the first separation step can be set to 30°C to 64°C in an embodiment or 50°C to 60°C in another embodiment. The reaction time can be set to 0.5 hours or more in an embodiment, 4 hours or more in another embodiment, or 8 hours or more in still another embodiment. During this period, it is preferred to stir the reaction liquid. The upper limit of the reaction time is not particularly limited, but can be, for example, within 20 hours in an embodiment or within 12 hours in another embodiment.

After the above reaction (or stirring) is completed, the reaction liquid is placed statically for 0.2 to 12 hours thereby to separate the first oil component, which contains a fatty acid alkyl ester, unreacted oil/fat, and the like, and the first glycerin-containing liquid, which contains an acidic glycerin phase and/or an inorganic salt. The first oil component is subjected to a further acid-catalyzed esterification reaction and can thereby be used for the generation of a fatty acid alkyl ester. On the other hand, the first glycerin-containing liquid is subjected to the subsequent neutralization step.

### (8) Neutralization Step

The neutralization step is a step of neutralizing the first glycerin-containing liquid, which is obtained in the first separation step, with an alkaline substance.

Examples of such an alkaline substance for use include hydroxides such as potassium hydroxide and sodium hydroxide.

Additionally or alternatively, a substance that contains glycerin can be used as the above alkaline substance. Examples of such a glycerin-containing alkaline substance include by-products obtained by the alkali-catalyzed transesterification reaction of oil/fat, such as the above waste glycerin. These can not only neutralize acidic glycerin but also increase the yield of glycerin, and therefore the use of a glycerin-containing alkaline substance is preferred also from such a perspective. The glycerin-containing alkaline substance may contain a fatty acid salt and/or a fatty acid glycerin ester.

The glycerin content in the above glycerin-containing alkaline substance is preferably 25 mass% or more and particularly preferably 50 mass% or more. The upper limit is not particularly limited, but may be, for example, 99 mass% or less in an embodiment or 90 mass% or less in another embodiment.

The pH of the above glycerin-containing alkaline substance is preferably 9 or more and particularly preferably 9 to 13.

Additionally or alternatively, a composition that contains a fatty acid salt as the main component may be used as the above alkaline substance. Examples of the alkaline substance containing a fatty acid salt as the main component include an oil cake and alkaline soap.

In the above neutralization step, the neutralization is preferably performed so that the pH of the glycerin-containing liquid becomes 4 to 8 in an embodiment, 4.5 to 7.5 in another embodiment, or 5 to 7.5 in a further embodiment. By neutralizing the glycerin-containing liquid so that the pH is within such a range, the oil component can be readily separated and the inorganic salt can be readily precipitated in the subsequent second separation step. The pH of the glycerin-containing liquid can be appropriately adjusted by controlling the additive amount of the above alkaline substance.

In the neutralization step, it is preferred to add the above alkaline substance while stirring the acidic glycerin-containing liquid so that the liquid property shifts from acidic to near neutral. As previously described, a substance that contains a fatty acid salt may be used as the alkaline substance used for neutralization, in which case the addition order as described above allows the fatty acid salt to be converted into a free fatty acid by the acid. The free fatty acid migrates from the glycerin-containing liquid to the oil phase which is phase-separated from the glycerin-containing liquid, and is less likely to redissolve into the glycerin-containing liquid even when the pH of the glycerin-containing liquid becomes high. This allows the separation in the subsequent second separation step to be easier. The fatty acid salt is contained not only in the above-described substance which contains a fatty acid salt as the main component, but also in a by-product of an alkali-catalyzed transesterification reaction or alkali hydrolysis of oil/fat.

The above alkaline substance neutralizes the first glycerin-containing liquid obtained in the first separation step. The neutralized glycerin-containing liquid is subjected to the subsequent second separation step.

### (9) Second Separation Step

The second separation step is a step of separating the second oil component and the precipitated inorganic salt from the neutralized glycerin-containing liquid, which is obtained in the neutralization step, and obtaining a second glycerin-containing liquid.

Here, the second oil component separated contains not only oil/fat and a fatty acid that are not separated even in the first separation step and remain in the first glycerin-containing liquid, but also oil/fat, a free fatty acid, and the like that are derived from the alkaline substance added in the neutralization step.

The inorganic salt separated in the second separation step is a salt of an inorganic acid (such as concentrated sulfuric acid) added in the first separation step and an alkali (such as potassium or sodium) and is preferably potassium sulfate. The above alkali is contained in the raw material (such as waste glycerin) charged in the first separation step and/or the alkaline substance added in the neutralization step, and the inorganic salt is precipitated in the first separation step and/or the neutralization step.

On the other hand, the glycerin-containing liquid contains not only glycerin but also a monohydric alcohol, water, and the like derived from the waste glycerin. The oil component and the inorganic salt have low solubility in the glycerin-containing liquid and therefore are separated from the glycerin-containing liquid.

In the second separation step, after the neutralized raw material is placed statically for about 3 to 12 hours, the upper liquid (oil component) and the lower liquid (glycerin-containing liquid) are separately recovered, and the glycerin-containing liquid can be obtained as the lower liquid, but it is preferred to increase the separation speed by centrifugal separation or the like. In such centrifugal separation, a three-phase separation type centrifugal separator may be preferably used, which can separate the upper liquid (i.e., an oil component), the lower liquid (i.e., a glycerin-containing liquid), and a solid substance (i.e., an inorganic salt). When a large amount of inorganic salt is precipitated, it is preferred to preliminarily separate a certain amount of inorganic salt using a centrifugal separator capable of solid-liquid separation, such as a decanter type centrifugal separator, and then further separate the liquid phase portion using a three-phase separation type centrifugal separator.

The second oil component obtained in the second separation step may be combined with the first oil component separated, for example, in the first separation step and subjected to a further acid-catalyzed esterification reaction (esterification step described later) and can thereby be used for the generation of a fatty acid alkyl ester. Specifically, the second oil component obtained in the second separation step can be used as a raw material for producing a fatty acid methyl ester (FAME) to be a biodiesel fuel. That is, methanol and a catalyst are added to the oil component to cause a methyl esterification reaction.

Additionally or alternatively, the inorganic salt can be used as a raw material for an inorganic fertilizer or the like through, for example, a washing step and the like.

On the other hand, the second glycerin-containing liquid obtained as described above can be used without any modification as a methane fermentation accelerator, but when removing a monohydric alcohol derived from the raw material or the like, the second glycerin-containing liquid may be further subjected to an alcohol removal step.

Being further subjected to an alcohol removal step may be preferred from the perspective of improving the efficiency of methane fermentation, from the perspective of workability, and from the perspective of avoiding the need to be treated as a dangerous substance. On the other hand, from the perspective of reducing the operation cost required for alcohol removal, or when the presence of the monohydric alcohol does not matter in the second glycerin-containing liquid (such as when the raw material does not contain monohydric alcohol and the second glycerin-containing liquid also does not contain monohydric alcohol, for example), the above second glycerin-containing liquid may be used as a methane fermentation accelerator without being subjected to the alcohol removal step.

### (10) Alcohol Removal Step

The alcohol removal step is a step of removing a monohydric alcohol (such as methanol) from the second glycerin-containing liquid obtained in the second separation step and is an optional step carried out as necessary.

The above second glycerin-containing liquid may contain a monohydric alcohol that is derived from the waste glycerin and remains in the first separation step (acid-catalyzed esterification reaction). The above second glycerin-containing liquid can be used as , a methane fermentation accelerator even when such a monohydric alcohol remains, but the efficiency in accelerating the methane fermentation can be improved by removing the monohydric alcohol.

In the alcohol separation/removal step, a reduced-pressure distillation method, a gas-liquid contact method, a membrane separation method, or other similar method can be adopted.

The reduced-pressure distillation method is a method in which the glycerin-containing liquid is heated (e.g., to about 60°C) to vaporize the monohydric alcohol such as methanol and then the pressure is reduced to separate the monohydric alcohol or the like. The separated monohydric alcohol or the like can be recovered by being cooled.

The gas-liquid contact method is a method in which the glycerin-containing liquid is brought into contact with the gas phase as fine droplets and the monohydric alcohol having a low boiling point is made to migrate to the gas phase for separation. Specifically, a spray-drying method or the like can be preferably adopted.

The membrane separation method is a method using a membrane that allows a monohydric alcohol to preferentially pass through.

The monohydric alcohol such as methanol recovered by distilling the second glycerin-containing liquid can be positively used in the production of biodiesel fuel.

The second glycerin-containing liquid may further contain water. Such water does not hinder the effect of accelerating the methane fermentation and may remain in the methane fermentation accelerator, but the water can be removed because it migrates to the gas phase together with the monohydric alcohol, for example, in the reduced-pressure distillation method, the gas-liquid contact method, or the like.

Additionally or alternatively, before or after the alcohol separation step of separating the above monohydric alcohol, further purification treatment may be performed using an ion-exchange method, activated white clay, diatomaceous earth, carbon, zeolite, or the like.

The monohydric alcohol separated in this step can be reused as a raw material for an alkali-catalyzed transesterification reaction or an acid-catalyzed esterification reaction without any modification or after being purified by redistillation or the like if necessary. Additionally or alternatively, the separated monohydric alcohol may be used as a washing liquid or the like for the inorganic salt or the like separated in the above second separation step.

The glycerin produced by the method according to the present embodiment has high purity and can be suitably applied to the methane fermentation accelerator according to the above-described embodiment. According to this production method, by going through the above-described first separation step, neutralization step, and second separation step, it is possible to obtain purified glycerin that can be applied to the methane fermentation accelerator, even though industrial waste such as waste glycerin can be used as a raw material, and the method is relatively simple.

The second glycerin-containing liquid obtained in the above method can be not only used as the methane fermentation accelerator, which will be described later, but also used for various applications such as a stripping agent for an asphalt-containing composition and a cement-containing composition; a denitrifying agent used as an organic carbon source in a biological nitrification/denitrification process; and an industrial raw material (e.g., a raw material for fatty acid glycerin ester). Additionally or alternatively, the second glycerin-containing liquid can be applied to applications requiring higher purity (e.g., cosmetics, foods and drinks, pharmaceuticals, etc.) after being subjected to a further step such as distillation.

### (11) Esterification Step

In the above-described first and second separation steps, the first and second oil components are respectively recovered from the separated oil phase. It is conceivable that these are circulated and supplied as raw materials in the production of a fatty acid alkyl ester by the alkali catalyst method, but the purity is not necessarily high; therefore, if they are used as the raw materials without any modification, it may be difficult to efficiently produce the fatty acid alkyl ester. Moreover, the first and/or second oil components contain oil/fat having a high acid value, such as a free fatty acid, and in particular, the first oil component is an acidic oil component because it is separated in the first separation step (first esterification step) which can be said as an esterification reaction using an acid catalyst. Thus, it becomes more difficult to use the first and second oil components without any modification as the raw materials for producing a fatty acid alkyl ester using an alkali catalyst.

Fortunately, however, a method other than the alkali catalyst method can produce a fatty acid alkyl ester even with oil/fat having a high acid value. In the present embodiment, therefore, it is preferred to provide an esterification step of producing a fatty acid alkyl ester by a method other than the alkali catalyst method.

In comparison with the previously described first separation step (first esterification step), this step may be referred to as a "second esterification step."

In the second esterification step, it is preferred to use the first oil component separated in the first separation step and/or the second oil component separated in the above second separation step as the raw materials.

The same raw materials (such as high acid value oil) as in the above acid reaction step (first esterification step) can be used as other raw materials.

By using these as the raw materials, it is possible to recycle industrial waste more efficiently in the above-described production of the methane fermentation accelerator. Any method other than the alkali catalyst method can be preferably used even with these raw materials.

In the second esterification step, it is preferred to use the monohydric alcohol separated in the above alcohol separation step as a raw material. This allows the industrial waste to be recycled more efficiently in the above-described production of the methane fermentation accelerator.

The method which can be adopted in the second esterification step is a method other than the alkali catalyst method, and more specifically, an acid catalyst method, an acid-alkali catalyst method, a biocatalyst method, an ion-exchange resin method, a supercritical method, a subcritical method, and a solid catalyst method are exemplified. These methods can perform a transesterification reaction with a monohydric alcohol such as methanol even for waste cooking oil or oil/fat having a high acid value or for oil/fat that contains an unreacted free fatty acid.

In the second esterification step, glycerin is produced as a by-product together with the oil component which contains a fatty acid alkyl ester. The oil component obtained in the second esterification step and the glycerin-containing liquid can be phase-separated by static placement, centrifugal separation, or the like. The separated oil component can be used as a biodiesel fuel or the like by recovering the fatty acid alkyl ester. On the other hand, the glycerin produced as a by-product can be supplied to the neutralization step, for example, together with the first glycerin-containing liquid obtained in the above first separation step (first esterification step). With such a configuration, the glycerin produced as a by-product in the second esterification step can be used as a part of the methane fermentation accelerator through the neutralization step, the second separation step, and the like, and can be further efficiently recycled.

It is particularly preferred to adopt, as the second esterification step, the acid catalyst method among the above-described methods other than the alkali catalyst method.

As illustrated in FIG. 2, when the acid catalyst method is adopted as the second esterification step, the above first oil component and/or second oil component may be used as the raw materials. Other raw materials for use may include the monohydric alcohol recovered in the alcohol removal step and the same raw materials (such as a high acid value oil) as in the first separation step (first esterification step).

The reaction liquid obtained in the second esterification step may be separated into an oil component that contains a fatty acid alkyl ester and a glycerin-containing liquid that contains glycerin produced as a by-product and an acid catalyst and its salt. Both the obtained oil component and the glycerin-containing liquid are acidic, among which the acidic glycerin-containing liquid can be supplied to the above neutralization step or the like.

On the other hand, it is preferred to perform neutralization, dehydration, or the like for the oil component which contains a fatty acid alkyl ester. Here, a method using waste glycerin produced as a by-product in the process of producing a biodiesel fuel is preferably exemplified as the method of neutralization/dehydration. Specifically, waste glycerin produced as a by-product in the process of producing the biodiesel fuel is dealcoholized and stored in a tank or the like, and the oil component to be neutralized is charged from the lower part of the tank to bring it into contact with the waste glycerin. This allows the acidic oil component to be neutralized by the alkali of the waste glycerin, and the water and monohydric alcohol contained in the oil component are absorbed by the waste glycerin liquid. Then, the oil component charged from the lower part overflows from the upper part due to the difference in specific gravity and can therefore be easily recovered. With such a method, neutralization, dehydration, and dealcoholization can be performed at the same time, and a high-quality oil component can be easily obtained. The waste glycerin liquid which has absorbed water and monohydric alcohol can be supplied to the above-described neutralization step and can be made into a part of the methane fermentation accelerator through the second separation step and the like.

In the second esterification step, a biocatalyst method, a supercritical method, and a subcritical method can be preferably exemplified as methods other than the acid catalyst method.

The biocatalyst method is a method of promoting the transesterification reaction using lipase or phospholipase having the catalytic activity for the transesterification reaction. The biocatalyst method has a characteristic that the reaction conditions are moderate, but the transesterification reaction can be accelerated even for oil/fat having a high acid value, and a by-product is less likely to be produced.

The supercritical method and the subcritical method are methods of promoting hydrolysis through adjusting the temperature and pressure to change the raw material into the supercritical state or subcritical state, thereby changing the phase state of a substance from two phases of gas and liquid to two phases of liquid and liquid, and further to one phase by lowering the dielectric constant, thus changing the reaction system, which originally requires the use of a catalyst, to a non-catalytic system.

By performing such a second esterification step, it becomes possible to recycle industrial waste further efficiently. The obtained fatty acid alkyl ester can be not only shipped as biodiesel fuel, bio-heavy oil, etc., but also subjected to power generation or the like thereby to recover energy. That is, there may be further provided a power generation step of generating electricity using the fatty acid alkyl ester obtained in the second esterification step.

The embodiments heretofore explained are described to facilitate understanding of the present invention and are not described to limit the present invention. It is therefore intended that the elements disclosed in the above embodiments include all design changes and equivalents to fall within the technical scope of the present invention.

### Examples

The present invention will be described below in more detail by illustrating production examples, testing examples, etc., but the present invention is not limited to the following examples and the like.

### [Production Example] Production of methane fermentation accelerator

### (Preparation of waste glycerin)

Biodiesel fuel was produced through transesterification between waste cooking oil and methanol by an alkali catalyst method using potassium hydroxide as the catalyst. The by-product containing glycerin generated at that time was recovered as waste glycerin.

To this waste glycerin, 20 g of zeolite was added per 1 kg of the waste glycerin to remove water. The zeolite-added waste glycerin was passed through a 250-mesh filter to remove the zeolite and solid impurities.

The composition and physical properties of the waste glycerin as a raw material thus obtained (referred to as "raw material waste glycerin," hereinafter) are as listed in Table 1.

**[Table 1]**

| | | |
|---|---|---|
| Composition (mass%) | Glycerin | 38 |
| | Fatty acid glycerin ester | 28 |
| | Fatty acid | 15 |
| | Potassium | 3 |
| | Methanol | 9 |
| | Water | 3 |
| Physical properties | Property | Black liquid |
| | pH | 8.9 |
| | Specific gravity (kg/liter) | 1.045 |

### (First separation step)

Into a reaction tank having a volume of 1,000 L (liters) and having a heating/cooling function, 500 kg of the raw material waste glycerin and 300 kg of high acid value oil (150 mgKOH/g) were charged, and they were heated to 55°C while being stirred (120 rpm). In this state, 32 L of concentrated sulfuric acid was added into the reaction container over 15 minutes. When adding the concentrated sulfuric acid, attention was made so that the temperature of the mixture in the reaction container would not exceed 65°C. The pH of the reaction liquid after the total amount of concentrated sulfuric acid was added was 1. After the addition of concentrated sulfuric acid was completed, stirring was continued for 240 minutes. After that, the mixture was placed statically for 10 hours to separate it into an oil phase (first oil component) and an acidic glycerin phase (first glycerin-containing liquid), and the first glycerin-containing liquid (acidic glycerin phase, including precipitated potassium sulfate) was recovered. By repeating the above operation, 5,000 kg of the first glycerin-containing liquid was obtained.

### (Neutralization step)

Into a reaction tank having a volume of 15,000 L, 5,000 kg of the first glycerin-containing liquid and 5,000 kg of waste glycerin were charged while being stirred. The pH was 7.1. After that, stirring was continued for 4 hours, and the mixture was then placed statically for 24 hours.

### (Second separation step)

The neutralized glycerin was treated with a decanter type centrifugal separator (product name: Z18H-V, available from TANABE WILLTEC INC.) at 5,500 rpm for 180 minutes, and the precipitated potassium sulfate was separated and recovered. The liquid phase was further treated with a three-phase separation type centrifugal separator (available from Alfa Laval AB) at 8,000 rpm for 180 minutes, and the second oil component, second glycerin-containing liquid, and potassium sulfate were separated and recovered.

### (Alcohol removal step)

The second glycerin-containing liquid obtained by the above second separation step was distilled in a batch system at a distillation temperature of 110°C for 10 minutes using a vacuum distillation apparatus to remove methanol and water. The obtained glycerin-containing liquid, containing 870,000 mg/kg of glycerin, was used as a methane fermentation accelerator.

### [Testing Example] Methane Fermentation Test

A methane fermentation test was conducted using the methane fermentation accelerator obtained in the production example and organic waste as follows. Dehydrated swine manure sludge obtained from a pig farm and excess dehydrated sludge obtained from a human waste treatment facility were used as organic waste, and they were mixed in the ratios listed in Table 1 to provide respective raw materials under test. The compounding ratios and properties of the raw materials under test are listed in Table 2.

The methane fermentation accelerator and organic waste were measured for pH, COD_{Cr}, T-N, T-P, n-Hex extract, and glycerin concentration. For the mixed raw materials under test, these values were calculated from the compounding ratios.

COD (chemical oxygen demand) refers to an index that represents an amount of organic substances in a composition as "oxygen consumption when decomposed by an oxidant," and there are multiple types of CODs depending on the type of oxidant used and the reaction conditions. In the present embodiment, "COD_{Cr}" using potassium dichromate as the oxidant can be preferably used as the COD index. This is because "COD_{Mn}" using potassium permanganate as the oxidant has a low capture rate with respect to the actual amount of organic substances. COD_{Cr} was measured in accordance with JIS K0102-20.2 "Absorbance method."

Then, T-N (total nitrogen) indicates the total amount of nitrogen compounds contained in a composition, and was measured in accordance with the "Kjeldahl nitrogen method" in Section 18-1 of Chapter 1, Volume 5 of the Sewage Testing Methods. T-P (total phosphorus) indicates the total amount of phosphorus compounds contained in a composition, and was measured in accordance with JIS K0102-46.3.2, "Nitric acid-perchloric acid decomposition method."

Next, n-Hex (n-hexane extract) was measured by the extraction/gravimetric method defined in Public Notice No. 64 of the Ministry of the Environment in 1974, Appendix Table 4. The n-Hex is a general term for non-volatile substances extracted with n-hexane, which is an organic solvent, and is used as an index representing the amount of "oil components and the like" in a composition. Here, the oil components and the like include animal/plant oil/fat; fatty acid; fatty acid ester; fatty acid derivatives such as phosphatide; wax; grease; and petroleum hydrocarbon.

The glycerin concentration was measured by liquid chromatography.

**[Table 2]**

| Table 2. Properties of raw materials under test | | | | | | | | |
|---|---|---|---|---|---|---|---|---|
| Sample No. | | | Sample 1 | Sample 2 | Sample 3 | Sample 4 | Sample 5 | |
| Mixing ratio | Mass ratio | Methane fermentation accelerator | 100% | 62% | 48% | 0% | 0% | |
| | | Swine manure | 0% | 38% | 30% | 100% | 58% | |
| | | Excess dehydrated sludge | 0% | 0% | 22% | 0% | 42% | |
| | COD_{Cr} ratio | Methane fermentation | 100% | 90% | 83% | 0% | 0% | |
| | | accelerator | | | | | | |
| | | Swine manure | | 0% | 10% | 10% | 100% | 60% |
| | | Excess dehydrated sludge | | 0% | 0% | 7% | 0% | 40% |
| Properties | pH | | - | 6.4 | 6.8 | 7.0 | 7.4 | 7.5 |
| | COD_{Cr} | | mg/kg | 1,100,000 | 761,800 | 632,800 | 210,000 | 201, 600 |
| | T-N | | mg/kg | 200 | 6,204 | 7,096 | 16,000 | 13,480 |
| | T-P | | mg/kg | 130 | 2,019 | 2,538 | 5,100 | 4,764 |
| | n-Hex extract | | mg/kg | 1,500 | 5,110 | 5,010 | 11,000 | 8,270 |
| | Glycerin | | mg/kg | 870,000 | 539,400 | 417, 600 | 0 | 0 |

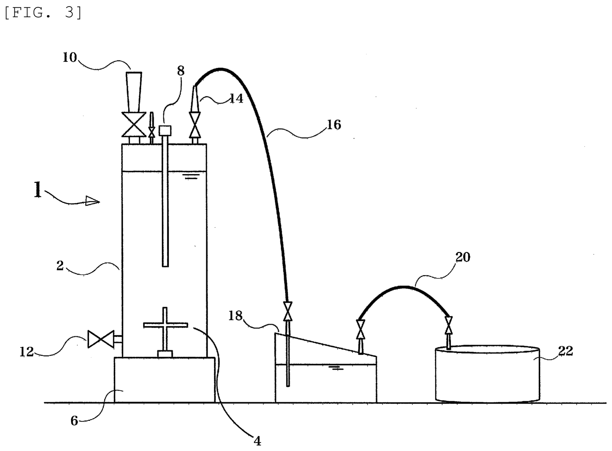

Then, for each of the raw materials under test, a methane fermentation experiment was carried out in the following manner using a methane fermentation apparatus illustrated in FIG. 3, and the amount of biogas generated from each sample was measured. The test was terminated when 26 days had passed from the start of the test (charge with raw materials).

The methane fermentation apparatus used in the examples will be described with reference to FIG. 3. The methane fermentation apparatus 1 includes a fermenter 2, a stirrer 4, a heating (and stirring) device 6, a thermometer 8, a raw material input port 10, a drain 12, and a gas outlet 14. The fermenter 2 is a sealed cylindrical tank made of SUS with a capacity of 3.5 L. A gas tube 16 is attached to the gas outlet 14 of the fermenter 2, and the gas tube 16 is connected to a gas flow meter 18 (volumetric flow meter). The flow meter 18 has a built-in data logging device, which enables monitoring and recording of the amount of biogas generated continuously for 24 hours. An aluminum gas pack 22 for collecting the generated biogas is also connected to the flow meter 18 via another gas tube 20.

First, 3.5 L of sludge acclimated with food residue containing nitrogen/phosphorus was charged into the fermenter 2 as seed sludge. The properties of the seed sludge were as follows: TS: 3.5%, COD_{Cr}: 34,000 mg/L, T-N: 5,100 mg/L, and T-P: 510 mg/L.

Then, each raw material under test in the amount listed in Table 3 was charged into the fermenter 2 while heating the inside of the fermenter to 37°C. The atmosphere in the fermenter 2 was purged with nitrogen, the stirrer 4 was constantly operated while keeping the temperature in the vessel at 37°C±0.5°C, and the inside of the fermenter 2 was continuously stirred. The amount of gas generated was measured using the flow meter 18, and the total amount of gas was collected by the gas pack 22 connected to the flow meter. Here, the amount of gas generated was continuously recorded by the data logger attached to the flow meter 18.

The methane concentration of the biogas collected in the gas bag 22 was measured by gas chromatography.

The results are listed in Table 3.

Here, the methane generation amount (unit: n-mL) in Table 3 refers to a value obtained through calculating an amount of methane gas obtained by multiplying the volume of the generated biogas by the methane concentration, and then converting it to standard conditions (0°C, 1 atm, 0% humidity). The amount of biogas generated was calculated by subtracting the amount of biogas generated (5,500 n-mL) measured in the blank (seed sludge only), and then converting it to the methane generation amount, as listed in Table 3.

The methane generation basic unit (n-m³/t) refers to a value obtained through converting the volume of methane generated (unit: m³) to standard conditions (0°C, 1 atm, 0% humidity) to obtain the methane generation amount (unit: n-m³), and dividing this by the mass of raw material (unit: t). decomposition COD_{Cr} (g) was calculated from the amount of methane generated using the formula: methane generation amount/decomposition COD_{Cr} = 0.35 n-m³/kg·COD_{Cr}. The COD_{Cr} decomposition ratio was calculated by dividing the decomposition COD_{Cr} by the COD_{Cr} (g) of the input raw material.

**[Table 3]**

| Table 3. Results | | | | | |
|---|---|---|---|---|---|
| Sample No. | Sample 1 | Sample 2 | Sample 3 | Sample 4 | Sample 5 |
| Raw material under test (g) | 50 | 50 | 50 | 100 | 100 |
| CH4 generation amount (n-m3/t) | 16, 305 | 13,385 | 9,899 | 3,989 | 3,170 |
| CH4 generation amount basic unit (n-m3/t) | 326 | 268 | 198 | 40 | 32 |
| Estimated CH4 generation amount (n-m3/t) | - | 217 | 173 | - | - |
| Input COD_{Cr} (g) | 55.0 | 38.1 | 31.6 | 21.0 | 20.2 |
| Decomposed COD_{Cr} (g) | 46.6 | 38.2 | 28.3 | 11.4 | 9.1 |
| COD_{Cr} decomposition ratio (%) | 84.7 | 100.4 | 89.4 | 54.3 | 44.9 |

As listed in Table 3, Sample 2, which was a mixture of dewatered swine manure sludge and a methane fermentation accelerator, had a methane generation basic unit of 268 nm-m³/t, which was greater than the estimated methane generation amount of 217 n-m³/t calculated from the mixing ratio, indicating significant acceleration of methane fermentation. Furthermore, Sample 2 had a significantly higher COD_{Cr} decomposition ratio than organic waste alone or the methane fermentation accelerator alone. Since the COD_{Cr} decomposition ratio exceeded 100%, it is believed that methane fermentation was carried out using the carbon source in the seed sludge in addition to the input raw material.

In addition, Sample 3, which was a mixture of dewatered swine manure sludge and excess dewatered sludge with a methane fermentation accelerator, had a methane generation ratio of 198 n-m³/t, which was greater than the estimated methane generation amount of 173 n-m³/t calculated from the mixing ratio, confirming that methane fermentation was also significantly accelerated with this methane fermentation raw material. Sample 3 also had a higher COD_{Cr} decomposition ratio than organic waste alone and the methane fermentation accelerator alone.

## Claims

1. A methane fermentation method comprising charging organic waste into a methane fermentation system to produce biogas containing methane,
wherein a methane fermentation accelerator is used in combination with the organic waste,
wherein the methane fermentation accelerator contains glycerin, wherein the methane fermentation accelerator contains an n-hexane extract of 10,000 mg/kg or less.

2. The methane fermentation method according to Claim 1, wherein the glycerin content in the methane fermentation accelerator is 600,000 mg/kg or more.

3. The methane fermentation method according to Claim 1 or 2, wherein the methane fermentation accelerator is used so that a ratio of COD_{Cr} in the methane fermentation accelerator to COD_{Cr} in the organic waste is 10:1 to 1:10.

4. A method for producing a methane fermentation accelerator to be combined with organic waste and charged into a methane fermentation system, comprising:
a first separation step of mixing a raw material containing at least one of glycerin and a fatty acid glycerin ester with an inorganic acid and separating a first oil component and a first glycerin-containing liquid;
a neutralization step of neutralizing the first glycerin-containing liquid with an alkaline substance; and
a second separation step of separating a second oil component and a precipitated inorganic salt from the neutralized first glycerin-containing liquid,
wherein the obtained methane fermentation accelerator contains glycerin.

5. The method for producing a methane fermentation accelerator according to Claim 4, further comprising
an alcohol removal step of removing monohydric alcohol after the second separation step.

6. The method for producing a methane fermentation accelerator according to Claim 4 or 5, wherein pH of a mixed liquid of the raw material and the inorganic acid in the first separation step is 3 or less.

7. The method for producing a methane fermentation accelerator according to any one of claims 4 to 6, wherein the first glycerin-containing liquid is neutralized to pH of 4 to 8 in the neutralization step.

8. The method for producing a methane fermentation accelerator according to any one of claims 4 to 7, wherein the methane fermentation accelerator contains an n-hexane extract of 10,000 mg/kg or less.

9. The method for producing a methane fermentation accelerator according to any one of claims 4 to 8, wherein the glycerin content in the methane fermentation accelerator is 600,000 mg/kg or more.
